Europäisches Patentamt

European Patent Office

Office européen des brevets

(19)

(11) EP 0 884 384 A2

## (12) EUROPEAN PATENT APPLICATION

(43) Date of publication:
16.12.1998 Bulletin 1998/51

(51) Int Cl.⁶: C12N 9/00

(21) Application number: 98250162.9

(22) Date of filing: 13.05.1998

(84) Designated Contracting States:
AT BE CH CY DE DK ES FI FR GB GR IE IT LI LU
MC NL PT SE
Designated Extension States:
AL LT LV MK RO SI

(30) Priority: 13.05.1997 JP 122635/97
07.05.1998 JP 125121/98

(71) Applicant: Ezaki Glico Co., Ltd.
Osaka-shi, Osaka 555-8502 (JP)

(72) Inventors:
• Terada, Yoshinobu
  Nishinomiya-shi, Hyogo (JP)
• Fujii, Kazutoshi
  Suita-shi, Osaka (JP)
• Yanase, Michiyo
  Kako-gun, Hyogo (JP)
• Takata, Hiroki
  Kobe-shi, Hyogo (JP)
• Takaha, Takeshi
  Kobe-shi, Hyogo (JP)
• Okada, Shigetaka
  Ikoma-shi, Nara (JP)

(74) Representative: UEXKÜLL & STOLBERG
Patentanwälte
Beselerstrasse 4
22607 Hamburg (DE)

### (54) Heat-resistant amylomaltase

(57)     The present invention relates to novel heat-resistant amylomaltase, a gene encoding the same, and a process for producing the same. The amylomaltase generates a cyclic glucan by an intramolecular transglycosylation reaction, using an α-glucan as a substrate, but does not substantially catalyze a hydrolytic reaction. The present invention also relates to a method for producing a cyclic glucan and a method for improving food using the heat-resistant amylomaltase.

FIG.1

**Description**

BACKGROUND OF THE INVENTION

1. FIELD OF THE INVENTION:

The present invention relates to novel heat-resistant amylomaltase, a gene encoding the same, and a process for producing the same. The present invention also relates to a method for producing a cyclic glucan and a method for improving food using the heat-resistant amylomaltase.

2. DESCRIPTION OF THE RELATED ART:

Amylomaltase (EC. 2.4.1.25) is an enzyme which is also called D-enzyme, 4-a-glucanotransferase, etc. This enzyme catalyzes the reaction of transferring an $\alpha$-glucan chain from one $\alpha$-glucan molecule to another (or glucose). Amylomaltase is widely distributed in microorganisms (e.g., *Escherichia coli* (hereinafter, referred to as "*E. coli*")), and plant tissues such as potato tubers, germinating barley seeds, sweet potato, and spinach. In general, enzymes derived from microorganisms are called amylomaltase, and enzymes derived from plants called D-enzyme. Amylomaltase derived from microorganisms and D-enzyme derived from plants have similarity in their amino acid sequences.

Recently, amylomaltase has been found to be capable of catalyzing a cyclization reaction of an $\alpha$-glucan. For example, it has been reported that amylomaltase is capable of catalyzing an intramolecular transglycosylation reaction (cyclization reaction) of amylose and synthesizing cycloamylose which is a cyclic $\alpha$-1,4-glucan having a degree of polymerization (hereinafter, referred to as a "DP") of 17 or more. It has also been reported that amylomaltase is capable of catalyzing a cyclization reaction of a branched $\alpha$-glucan such as amylopectin, and synthesizing a branched cyclic glucan (see Japanese Laid-open Publication No. 8-311103). By utilizing this reaction, branched cyclic glucans having various structures such as an inner branched cyclic glucan and an outer branched cyclic glucan can be synthesized.

Such a cyclic glucan has the ability to form an inclusion complex and has a very high solubility in water. Therefore, it has been expected that the cyclic glucan may be applied to food, pharmaceutical preparations, etc. The cyclic glucan is useful, in particular, as a raw material in the starch processing industries, food and drink compositions, food additive compositions, infusion solutions, adhesive compositions, inclusion materials or adsorption materials, anti-retrogradation agents for starch, or starch substitutes for biodegradable plastics. (see Japanese Laid-open Publication No. 8-311103).

Hithertofore, the presence of amylomaltase (D-enzyme) derived from plants has been recognized in various plant tissues such as potato tubers, germinating barley seeds, roots of sweet potato, and green leaves of spinach. Regarding amylomaltase derived from a potato, cDNA has been isolated, and its nucleotide sequence has been determined (Takaha et al., J. Biol. Chem. Vol. 268, pp. 1391-1396 (1993)). Amylomaltase derived from a potato has been expressed in *E. coli,* while maintaining its activity, and has been utilized for producing cycloamylose on a laboratory scale (Takaha et al., *J.* Biol. Chem. Vol. 271, pp. 2902-2908, (1996)).

On the other hand, regarding amylomaltase derived from microorganisms, an enzyme derived from *E. coli,* which is a mesophilic bacterium, has well been studied (Eur. J. Biochem. Vol. 69, pp. 105-115, (1976)). Furthermore, a nucleotide sequence of an amylomaltase gene derived respectively from *Haemophilus influenzae, Streptococcus pneumoniae,* and *Clostridium butyricum,* which are mesophilic bacteria, has been determined (GenBank Access No. U32760, J01796, L37874).

Amylomaltase can be applied for various industrial purposes. As an example, amylomaltase can be utilized in processing an $\alpha$-glucan such as the production of a cyclic glucan. In the case of using an enzyme for an industrial purpose, the reaction is desirably conducted at a temperature as high as possible (about 60°C or higher). This is because an $\alpha$-glucan which is a substrate is prevented from retrogradation, and a reaction system is prevented from the contamination of microorganisms. However, among the above-mentioned known amylomaltase, the enzymes whose properties are known have high activity in a moderate temperature range of about 30°C to about 45°C. For example, the reaction optimum temperature of amylomaltase derived from *E. coli* is about 35°C (Agric. Biol. Chem. Vol. 53, pp. 2653-2659, (1989)), the reaction optimum temperature of amylomaltase derived from a potato is about 45°C (J. Chem. Soc., pp. 44-53, (1956)). On the other hand, regarding the amylomaltase of *Haemophilus influenzae, Streptococcus pneumoniae,* and *Clostridium butyricum,* the purification and various properties of these enzymes have not been studied. However, it is assumed from the growth temperature of these bacteria that the optimum temperature of these enzymes is also in a moderate temperature range of about 30°C to about 45°C. Thus, known amylomaltase is substantially limited to the enzymes which have a high activity in a moderate temperature range. Since it is difficult to use these enzymes for the reaction at a high temperature of about 60°C or higher, these enzymes are not suitable for use in the processing of an $\alpha$-glucan on an industrial scale.

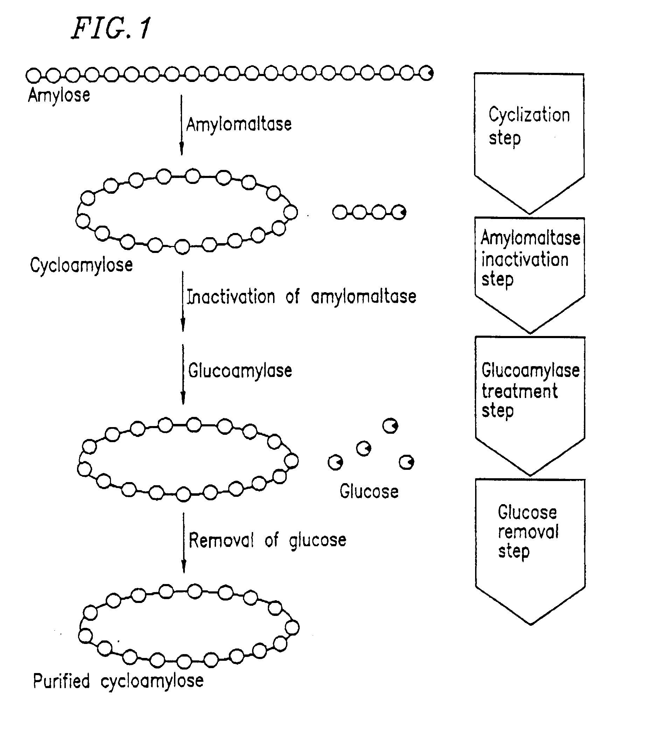

Furthermore, amylomaltase is known to catalyze the reaction of cleaving a cyclic glucan and transferring it to an acceptor molecule (i.e., ring-opening reaction), thereby degrading the cyclic glucan in the presence of an acceptor such as glucose, as well as catalyzing the cyclization reaction of an α-glucan chain (Takaha et al., J. Biol. Chem. Vol. 271, pp. 2902-2908 (1996)). Thus, with reference to the process for producing a cyclic glucan shown in Figure 1, if active amylomaltase remains in the step of treatment with glucoamylase in which a great amount of glucose is generated, the degradation of the cyclic glucan proceeds, resulting in a large decrease in yield of the cyclic glucan. Therefore, in order to effectively produce the cyclic glucan, it is desirable that amylomaltase is completely inactivated after the completion of the cyclization step and prior to the step of treatment with glycoamylase. Considering the application for industrial purposes, as the method for inactivating an enzyme, heating inactivation is preferable. More practically, it is preferable that an enzyme is completely inactivated by being held at a temperature of about 90°C to about 100°C for several minutes. Therefore, enzymes having higher heat-resistance than necessary, such as amylomaltase possibly being isolated from archaebactria or archaea which have an extraordinarily high optimum growth temperature (i.e., hyper thermophiles) are not suitable.

Furthermore, amylomaltase such as that derived from *E. coli* is known to have a hydrolytic activity. Therefore, not only a linear glucan which is a substrate for the cyclization reaction but also a cyclic glucan as a product are hydrolyzed by the action of the enzyme. As a result, there is a problem that the yield of the cyclic glucan, which is the product of interest, decreases.

As described above, the problem for the effective production of cyclic glucans, while preventing the contamination of microorganisms and the retrogradation of α-glucan, has not sufficiently been solved. Therefore, there has been a demand for amylomaltase suitable for the production of a cyclic glucan, which has heat-resistance in the reaction temperature at about 60°C, is easy to inactivate after the reaction, and does not substantially catalyze the hydrolytic reaction.

Furthermore, as another example of applying amylomaltase for industrial purposes, amylomaltase can be directly used to improve food containing starch in large amount.

Starch is the major component of cereals and is included in significant amount in various foods. However, conventional starch have several undesirable properties. Starch has very low solubility in water, and has very high viscosity when gelatinized. Furthermore, dissolved or gelatinized starch is retrograded rapidly and change the physical properties of starch solution or gelatinized starch. This retrogradation of starch sometimes cause a serious problem in foods containing significant amount of starch. Such foods lost their original properties, such as texture, water holding property, shape holding property, resistance against freezing, digestibility and the like. Therefore, there has been a strong demand for a technique of suppressing the retrogradation of starch in food, and in the past, two methods have been used.

The first method is to add a substance having an effect to prevent the retrogradation of starch into food. Hithertofore, a number of substances, such as saccharides, polysaccharides, sugar alcohols, proteins, and fatty acid esters, have been employed for this purpose. As described above, it has also been reported that the retrogradation of starch was prevented by adding a cyclic glucan to starch (see Japanese Laid-Open Publication No. 8-311103).

The second method is to diminish the molecular size of starch by directly adding an enzyme which acts on starch to food. An enzyme used in this method is mainly amylase which hydrolyzes starch. For example, a method using heat-resistant β-amylase (see Japanese Laid-Open publication Nos. 62-79745 and 62-79746) and a method using maltogenic α-amylase (see European Patent Publication No. 494 233) have been reported. However, amylase is an hydrolase which generates a low molecular weight saccharide having a sweet taste, so that its use is limited. Thus, amylase is insufficient for improving food containing starch.

Glucanotransferases such as amylomaltase have also employed for this purpose (e.g., see PCT international publication NO. WO97/41735), since the molecular size of starch is diminished by a cyclization reaction, and a cyclic glucan generated have the effect to prevent the retrogradation of starch. However, most of the known amylomaltase is not suitable for this application, since most of such enzyme also catalyze the hydrolysis of starch and accumulate reducing sugars with low molecular weight. Furthermore, enzymes whose optimum temperature is in a modest temperature range of about 30°C to about 45°C cannot conveniently be used, because they are inactivated at a higher temperature during cooking of a food material by heating.

Therefore, there has been a demand for heat-resistant amylomaltase which does not substantially catalyze a hydrolytic reaction, and thus can be used to improve food.

## SUMMARY OF THE INVENTION

The present inventors isolated a novel class of amylomaltase from Thermus Flavus ATCC 33923 strain. This enzyme has particularly outstanding properties for producing a cyclic glucan and improving food among the same kind of enzymes previously found. The inventors completed the present invention based on this finding.

Amylomaltase of the present invention has the following properties: 1) function: generating a cyclic glucan by an intramolecular transglycosylation reaction, using an α-glucan as a substrate; 2) reaction specificity: not substantially catalyzing a hydrolytic reaction; 3) reaction optimum temperature: 65°C to 70°C; 4) heat-resistance: maintaining activity at 60°C for at least 10 minutes and being inactivated at 100°C in 15 minutes; and 5) reaction optimum pH: pH 5.5.

In one embodiment of the present invention, the above-mentioned amylomaltase is derived from Thermus flavus ATCC 33923.

Alternatively, amylomaltase of the present invention is a) or b) stated as follows: a) amylomaltase having the amino acid sequence from Met at 1st position to Leu at 500th position represented in SEQ.ID NO. 1 of Sequence Listing; or b) amylomaltase with heat-resistance, having an amino acid sequence in which one or more amino acids of a) are subjected to deletion, substitution, or addition.

According to another aspect of the present invention, a gene of the present invention encodes amylomaltase which is a) or b) stated as follows: a) amylomaltase having the amino acid sequence from Met at 1st position to Leu at 500th position represented in SEQ.ID NO. 1 of Sequence Listing; or b) amylomaltase with heat-resistance, having an amino acid sequence in which one or more amino acids of a) are subjected to deletion, substitution, or addition.

Alternatively, an amylomaltase gene of the present invention has DNA which is a) or b) stated as follows: a) DNA having a nucleotide sequence represented in SEQ.ID NO. 2 of Sequence Listing; or b) DNA which hybridizes to DNA of a) under stringent conditions, and encodes a heat-resistant protein having amylomaltase activity.

According to another aspect of the present invention, an expression vector containing the above-mentioned heat-resistant amylomaltase gene is provided.

According to another aspect of the present invention, a microorganism transformed with the above-mentioned expression vector is provided.

According to another aspect of the present invention, a method for producing amylomaltase of the present invention includes the steps of: culturing the above-mentioned transformed microorganism, and collecting and purifying amylomaltase produced by the culture.

According to another aspect of the present invention, amylomaltase produced by the above-mentioned method is provided.

According to another aspect of the present invention, a method for producing a cyclic glucan includes the steps of cyclizing an α-glucan using the above-mentioned amylomaltase, and collecting and purifying the cyclic glucan.

In one embodiment of the present invention, the cyclic glucan includes a cyclic α-1,4-glucan.

In another embodiment of the present invention, the cyclic glucan includes a branched cyclic glucan.

In another embodiment of the present invention, a branching enzyme is further used in the step of cyclization.

According to another aspect of the present invention, a method for producing food includes the step of adding the above-mentioned amylomaltase to a food material before or immediately after cooking by heating, wherein the amylomaltase acts on a starch in the food material to produce a cyclic glucan.

In one embodiment of the present invention, the food is selected from the group consisting of rice products, Japanese desserts, snacks, wheat products, noodles, gyoza skins, shumai skins, processed seafoods, frozen or refrigerated processed foods, baby foods, weaning foods, pet foods, animal feeds, drinks, sports foods, and nutrient supplemental foods.

According to another aspect of the present invention, a food material, a food additive composition, and a food improving agent, containing the above-mentioned amylomaltase are provided.

Thus, the invention described herein makes possible the advantages of (1) providing amylomaltase which has heat-resistance suitable for producing a cyclic glucan and does not substantially catalyze a hydrolytic reaction; (2) providing a gene encoding amylomaltase, allowing the amylomaltase to be produced recombinantly; (3) providing a method for producing a cyclic glucan with high efficiency and high yield, using the amylomaltase; (4) providing a method for producing improved food by adding the amylomaltase to a food material before or immediately after cooking by heating to diminish the molecular size of starch and to generate a cyclic glucan; and (5) providing a food material, a food additive composition, and a food improving agent containing the amylomaltase for improving food.

These and other advantages of the present invention will become apparent to those skilled in the art upon reading and understanding the following detailed description with reference to the accompanying figures.

BRIEF DESCRIPTION OF THE DRAWINGS

Figure 1 is a schematic diagram showing the steps for producing cycloamylose using amylomaltase.

Figure 2 is a photograph showing the result obtained by subjecting purified amylomaltase of the present invention to SDS polyacrylamide electrophoresis.

Figure 3 is a graph showing a reaction optimum temperature of the amylomaltase of the present invention.

Figure 4 is a graph showing an optimum pH of the amylomaltase of the present invention.

Figure 5 is a graph showing heat-resistance of the amylomaltase of the present invention, measured as the residual activity of the amylomaltase after heated at the designated temperatures for 10 minutes in a buffer A (see Examples 1 and 2).

Figure 6 is a photograph showing the result of thin layer chromatography studying a disproportionating reaction

EP 0 884 384 A2

of the amylomaltase of the present invention.

Figure 7 is a schematic diagram showing the steps of producing an amylomaltase expression vector.

Figure 8 is a graph obtained by monitoring the production of cycloamylose from amylose with time, using the amylomaltase of the present invention (closed circles) and amylomaltase derived from *E. coli* (open circles).

Figure 9 is a graph showing the results obtained by analyzing cycloamylose produced by using the amylomaltase of the present invention by a carbohydrate analysis system manufactured by DIONEX.

Figure 10 is a graph showing elution patterns on gel filtration chromatography of the waxy corn starches before (0h) and after (6h) treatment with the amylomaltase of the present invention.

Figure 11 is a schematic diagram showing the steps for analyzing the structure of cyclic glucans (see, Example 7 (B3)).

Figure 12 is a graph showing that a cyclic glucan was produced in bracken rice cake with an enzyme added (B). Bracken rice cake without an enzyme added was used as a control (A).

DESCRIPTION OF THE PREFERRED EMBODIMENTS

Hereinafter, the present invention will be described in detail.

The term "$\alpha$-glucan" as used herein refers to an $\alpha$-1,4-glucan (polysaccharide having a chain structure containing maltose as a constitutive disaccharide unit) or an $\alpha$-1,4-glucan having an $\alpha$-1,6-branch structure. Examples of the $\alpha$-glucan include amylose, amylopectin, starches, glycogen, waxy starches, high amylose starches, soluble starches, dextrins, hydrolyzed products from starches, and amylopectins enzymatically synthesized with phosphorylase.

The term "cyclic glucan" as used herein includes a cyclic $\alpha$-1,4-glucan having only $\alpha$-1,4-glucosidic bonds, and a branched cyclic glucan having both of an $\alpha$-1,4-glucosidic bond and an $\alpha$-1,6-glucosidic bond. The term "branched" refers to having at least one glucosidic bond other than the $\alpha$-1,4-bond. Examples of the branched cyclic glucan include an inner branched cyclic glucan containing a branch structure having an $\alpha$-1,6-bond in a cyclic structure, and an outer branched cyclic glucan containing a non-cyclic structure portion in addition to a cyclic structure.

The term "cycloamylose (CA)" as used herein refers to a cyclic $\alpha$-1,4-glucan having a DP of 17 or more.

The term "amylomaltase activity" as used herein refers to activity measured by quantitating glucose generated when amylomaltase is acted in a composition solution containing 100 milli mole (mM) of sodium acetate (pH 5.5) and 0.2% weight per volume (w/v) of maltotriose at 70°C for 10 minutes. The activity in which 1 micro mole ($\mu$M) of glucose is generated per minute is defined to be one unit.

The term "does not substantially catalyze a hydrolytic reaction" as used herein means that the yield of cycloamylose is not decreased from the highest yield, in the case where 0.07 unit/ml of amylomaltase is acted at 70°C for 6 hours in a buffer (100 mM of sodium acetate (pH 5.5), including 9% volume per volume (v/v) dimethyl sulfoxide (DMSO)) containing a final concentration of 0.2% (w/v) of enzymatically synthesized amylose AS-320.

The term "quantitation of cycloamylose" as used herein refers to quantitation of cycloamylose in an reaction product obtained by allowing amylomaltase to act on amylose. The amount of cycloamylose is measured as an amount of glucan which is not degraded by glucoamylase when the reaction product is digested by the enzyme.

The term "yield of cycloamylose" as used herein refers to the amount of quantitated cycloamylose calculated as a weight ratio with respect to a substrate.

The term "maintain activity at 60°C for 10 minutes or more" as used herein means that a decrease in activity is not observed in the case where amylomaltase is treated at 60°C for 10 minutes in a buffer A (10 mM of $KH_2PO_4$-$Na_2HPO_4$, pH 7.0). The term "inactivated at 100°C in 15 minutes" means that activity is not observed in the case where amylomaltase is treated at 100°C for 15 minutes in a buffer A (10 mM $KH_2PO_4$-$Na_2HPO_4$, pH 7.0).

The term "reaction optimum pH" as used herein refers to the pH at which amylomaltase activity becomes highest in the case where the amylomaltase is reacted at 70°C for 10 minutes at each pH in the presence of 0.2% (w/v) of maltotriose in a Britton-Rhobinson buffer.

The term "reaction optimum temperature" as used herein refers to the temperature at which amylomaltase activity becomes highest in the case where the amylomaltase is reacted at each temperature for 10 minutes (pH 5.5) in the presence of 0.2% (w/v) maltotriose in a buffer (100 mM of sodium acetate (pH 5.5)).

The term "thermophilic bacteria" as used herein refers to microorganisms whose growth optimum temperature is about 50°C to about 105°C and which hardly grow at about 30° or less. Among them, the microorganisms whose growth optimum temperature is 90°C or higher are called "hyper thermophiles".

The term "mesophilic bacteria" as used herein refers to microorganisms whose growth temperature is in an ordinary temperature environment, particularly, to microorganisms whose growth optimum temperature is about 20°C to about 40°C.

5

(Amylomaltase and purification thereof)

The amylomaltase of the present invention is heat-resistance, as described above. This enzyme maintains its activity, preferably at 70°C, for 10 minutes, more preferably at 80°C, for 10 minutes, and is inactivated, preferably at 100°C, in 10 minutes, more preferably at 100°C, in 5 minutes. Furthermore, this enzyme does not substantially catalyze a hydrolytic reaction, preferably even at 70°C.

Amylomaltase having an amino acid sequence represented in SEQ.ID NO. 1 of Sequence Listing and amylomaltase containing a deletion, a substitution, or an addition by one or more amino acid in the amino acid sequence represented in SEQ.ID NO. 1 of Sequence Listing are enzymes contemplated by the present invention. The amylomaltase containing a mutation has activity equal to or more than that of the amylomaltase not containing a mutation, heat-resistance similar to that of the amylomaltase not containing a mutation, and does not substantially catalyze a hydrolytic reaction.

The above-mentioned mutation can be one which occurs naturally, or can be generated by the action of a mutagen or by the artificial introduction of site-specific mutation. A procedure of a site-specific mutagenesis is well-known in the art. For example, see Nucl. Acid Research, Vol. 10, pp. 6487-6500 (1982). The term "a deletion, a substitution, or an addition by one or more amino acids" as used herein refers to a deletion, a substitution, or an addition of the number of amino acids which can be introduced by site-specific mutagenesis. This number is typically not more than about 15 amino acids, preferably not more than about 10 amino acids, and more preferably not more than about 5 amino acids, and still more preferably not more than about 3 amino acids. It is easy for those skilled in the art to select an amylomaltase mutant having desired properties.

The amylomaltase of the present invention can be obtained as follows. Thermophilic bacteria producing amylomaltase, such as Thermus Flavus ATCC 33923 strain are cultured in a large amount. Thereafter, the cells are destroyed, and centrifuged or filtered to separate destroyed cells, whereby a supernatant is obtained as a crude enzyme solution. Furthermore, the crude enzyme solution is subjected to an appropriate combination of ordinary methods for purifying an enzyme, such as dialysis, lyophilization, isoelectric focusing, ion exchange chromatography, and crystallization, whereby a purified enzyme with a higher specific activity can be obtained.

(Amylomaltase gene)

The gene encoding the amylomaltase of the present invention is a gene contemplated by the present invention. This gene can be obtained by a method known to those skilled in the art, based on the disclosure of the present specification.

For example, purified amylomaltase derived from Thermus Flavus ATCC 33923 strain is treated with trypsin. The digested fragments thus obtained is separated by HPLC, and an N-terminus sequence of a peptide fragment corresponding to one of the resulting peaks is identified by a peptide sequencer. A synthetic oligonucleotide probe is prepared based on the identified sequence. Then, an appropriate genomic library or a cDNA library is screened by using the oligonucleotide probe, whereby the amylomaltase gene of the present invention can be obtained. The basic strategy for preparing an oligonucleotide probe and DNA libraries and for screening them by nucleic acid hybridization is well-known to those skilled in the art. For example, see Sambrook et al., Molecular Cloning: A Laboratory Manual (1989); DNA Cloning, Vol. I and II (D.N. Glover ed., 1985); Oligonucleotide Synthesis (M. J. Gait ed., 1984); Nucleic Acid Hybridization (B.D. Hames & S.J. Higgins eds., 1984).

In the case of screening a genomic library, the gene thus obtained can be sub-cloned by using a method well-known to those skilled in the art. For example, a λ phage containing a gene of interest, an appropriate E. *coli* strain, and an appropriate helper phage are mixed, whereby a plasmid containing a gene of interest can easily be obtained. Thereafter, an appropriate E. *coli* strain is transformed by using a solution containing the plasmid, whereby the gene of interest can be sub-cloned. The transformant thus obtained is cultured and the plasmid DNA is extracted, for example, by an alkali SDS method. Using the extracted plasmid DNA, a nucleotide sequence of the gene of interest can be sequenced. A method for sequencing a nucleotide sequence is well-known to those skilled in the art. Furthermore, an amylomaltase gene can be amplified directly by a polymerase chain reaction (PCR), using a primer synthesized based on the nucleotide sequence of the amylomaltase gene. Genomic DNA of Thermus Flavus ATCC 33923 strain, for example, serves as a template for PCR.

A gene comprising DNA having the nucleotide sequence represented in SEQ.ID NO. 2 of Sequence Listing and a gene comprising DNA which hybridizes to DNA having the nucleotide sequence represented in SEQ.ID NO. 2 of Sequence Listing under stringent conditions are those contemplated by the present invention. Amylomaltase encoded by the latter gene has activity equal to or higher than that of Amylomaltase encoded by the former gene, heat-resistance similar to that of Amylomaltase encoded by the former gene, and does not substantially catalyze a hydrolytic reaction. The latter gene has typically not less than about 70% homology, preferably not less than about 80% homology, more preferably not less than about 90% homology, still more preferably not less than about 95% homology with respect to the former gene. It is easy for those skilled in the art to select a desired amylomaltase gene based on the disclosure of SEQ. ID No.2 of Sequence Listing.

The term "stringent conditions" as used herein refers to conditions under which a gene hybridizes with specific sequence but does not hybridize with a nonspecific sequence. The setting of the stringent conditions is well-known to those skilled in the art, for example, that which is described in Molecular Cloning (Sambrook et al., supra).

(Recombinant expression of amylomaltase)

Expression of a cloned gene is accomplished by constructing an appropriate expression vector in which cloned DNA is inserted, introducing the expression vector into a microorganism, culturing a recombinant microorganism, as well as recovering a product from the recombinant microorganism. These methods are well-known to those skilled in the art.

Microorganism hosts used in the present invention include procaryotes and eukaryots. Preferable procaryotic hosts include a mesophilic bacteria such as *E. coli.*

An expression vector refers to a medium in which a gene of interest is operably linked so that it is capable of being transcribed and translated. The medium may have additional elements required for replication in a microorganism and selection of a recombinant, if required. In the case where an expression product is intended to be secreted, a nucleotide sequence encoding a secretory signal peptide is linked in reading frame to DNA encoding a protein of interest at the upstream of the DNA. It is well-known to those skilled in the art that the kind of an appropriate expression vector can be varied depending upon a microorganism host to be used.

In some cases, an amylomaltase gene of interest should be processed so as to be operably linked to elements required for transcription and translation in the above-mentioned expression vector. Examples include the case where it is expected that a transcription efficiency would decrease since the distance between a promoter and a coding region is too long, and the case where an interval between a ribosome binding site and a translation initiation codon is not appropriate. Examples of the processing method include digestion with a restriction enzyme, digestion with exonuclease such as Bal31 and ExoIII, as well as introduction of a site-specific mutagenesis, using PCR or single-stranded DNA such as M13.

Appropriate conditions are selected for culturing a transformant which has obtained the ability of producing amylomaltase, as a result of the introduction of an expression vector. These conditions depend upon the kind of a host microorganism used and a factor for regulating expression in an expression vector, and a material to be expressed. For example, an ordinary shake culture method can be used.

A culture medium to be used is not particularly limited, as long as it allows a host microorganism to be used to grow. The culture medium may be contain a carbon source and a nitrogen source. It may also contain a salt such as an inorganic salt (e.g., phosphoriate, $Mg^{2+}$, $Ca^{2+}$, $Mn^{2+}$, $Fe^{2+}$, $Fe^{3+}$, $Zn^{2+}$, $Co^{2+}$, $Ni^{2+}$, $Na^{+}$, and $K^{+}$) as used alone or in an appropriate combination thereof, if required. Furthermore, various inorganic substances and organic substances required for growing a transformant and producing an enzyme can be added, if required.

The temperature for culturing a transformant to be used can be selected so as to be suitable for growing the transformant. It is usually 15°C to 60°C. The culture of the transformant strain is continued for a sufficient period of time to produce amylomaltase.

In the case of using an expression vector having an inducible promotor, the expression can be controlled by the addition of an inducible material, the alteration of the temperature for culture, the adjustment of components of a culture medium, etc. For example, in the case of using an expression vector having a lactose inducible promotor, the expression can be induced by adding isopropyl-1-thio-$\beta$-D-galactoside (IPTG).

After culturing the transformant as described above, in the case where, for example, an expression product is secreted outside the cells, the cultures are subjected to centrifugation or filtration to separate cells, whereby a supernatant is obtained. Next, the supernatant containing the amylomaltase is concentrated by using an ordinary method (e.g., salting-out method, solvent precipitation, ultrafiltration), and a fraction containing amylomaltase is obtained. This fraction is subjected to filtration, centrifugation, demineralization, etc., to obtain a crude enzyme solution. Furthermore, the crude enzyme solution is subjected to an appropriate combination of ordinary methods for purifying an enzyme, such as lyophilization, isoelectric focusing, ion exchange chromatography, and crystallization, whereby a purified enzyme or a crude enzyme having a higher specific activity can be obtained. If the enzyme thus obtained does not contain an enzyme which hydrolyzes an $\alpha$-glucan such as $\alpha$-amylase, the crude enzyme can be directly used for producing a cyclic glucan.

According to the above-mentioned recombinant expression, the productivity of the amylomaltase of the present invention can be largely improved. Furthermore, In the case where recombinant expression of the amylomaltase of the present invention is used, the amylomaltase can easily be purified by utilizing its heat-resistance. Specifically, by heating a cell extract containing amylomaltase at about 60°C, an enzyme contaminating the extract is made insoluble. This insoluble substance can be removed by centrifugation and subjected to dialysis to obtain the purified amylomaltase.

(Cyclization reaction by amylomaltase)

(1) Production of cyclic α-1,4-glucan

Cycloamylose which is a cyclic α-1,4-glucan can be produced by using the amylomaltase of the present invention and using an α-glucan as a substrate. In this case, amylomaltase can be used as an immobilized enzyme.

Cycloamylose can be produced by allowing amylomaltase to act in a buffer containing an appropriate concentration of an α-glucan. As the α-glucan, a straight chain α-1,4-glucan is preferable, and amylose with a DP of at least 20 or more is more preferable. The cyclization reaction smoothly proceeds by using the amylomaltase of the present invention, and typically, cycloamylose having a DP of about 20 to about 400 can be produced. As a main product, it is preferable that cycloamylose having a DP of about 20 to about 200 is produced.

The reaction temperature is 60°C or higher, preferably 60°C to 80°C, more preferably 65°C to 70°C. Thus, the amylose as a substrate can be prevented from being retrograded, and the reaction system can be prevented from the contamination of microorganisms.

After the cyclization reaction sufficiently proceeds, amylomaltase is completely inactivated at a high temperature, whereby a reverse reaction during a purifying step can be prevented. Preferably, the inactivating temperature is 90°C to 100°C.

Cycloamylose is recovered from the reaction solution and purified. In order to facilitate the removal of an non-cyclic glucan, after amylomaltase is inactivated, glucoamylase is added to the reaction solution, whereby an non-cyclic glucan can be degraded into glucose. Pure cycloamylose can be obtained by an ordinary purifying method such as ethanol precipitation or chromatography.

The enzyme of the present invention does not substantially catalyze a hydrolytic reaction. Therefore, the yield of cycloamylose can be maintained at a high level for a long period of time during the cyclization reaction. No decrease in the yield is observed from the beginning of the reaction until at least 6 hours later under the representative reaction conditions disclosed herein. Thus, the reaction with high yield can be performed with good reproducibility. According to the method of the present invention, cycloamylose can be produced with a yield of at least about 70%, typically about 75% to about 85%.

(2) Production of a branched cyclic glucan

Furthermore, a cyclization reaction of an α-glucan comprising a branch structure having an α-1,6-bond, such as amylopectin is catalyzed by the amylomaltase of the present invention, whereby branched cyclic glucans containing various structures such as an inner branched cyclic glucan and an outer branched cyclic glucan can be produced. In this case, amylomaltase can be used as an immobilized enzyme.

A branched cyclic glucan can be produced by allowing amylomaltase to act in a buffer solution containing an α-glucan in an appropriate concentration. As an α-glucan, an α-glucan containing a branch structure having an α-1,6-bond is preferable, and amylopectin, glycogen, starch, waxy starches are more preferable. By using the amylomaltase of the present invention, a cyclization reaction smoothly proceeds, and typically, a branched cyclic glucan having a DP of about 17 to about 5000 can be produced. In particular, it is preferable that a branched cyclic glucan having a DP of from about 21 to about 3000 is produced.

Furthermore, in order to produce a branched cyclic glucan having a more complicated structure, the amylomaltase of the present invention can be used together with an additional enzyme. For example, a branching enzyme (EC 2.4.1.18) can be used with amylomaltase. A branching enzyme is also called an α-1,4-glucan branching enzyme, which catalyzes a reaction of transferring a part of an α-1,4-glucan chain onto the 6-position of a glucose residue to produce a branch (e.g., see European Patent Publication No. 418 945). A more complicated branched cyclic glucan can be produced by using two or more enzymes.

The reaction temperature is 60°C or higher, preferably 60°C to 80°C, and more preferably 65°C to 70°C. This prevents α-glucan which is a substrate from being retrograded, and a reaction system from the contamination of microorganisms.

The amylomaltase of the present invention acts on an α-glucan such as amylopectin to produce a mixture of a branched cyclic glucan and a cyclic α-1,4-glucan. For purifying only the branched cyclic glucan from the mixture, any methods well-known to those skilled in the art can be used. For example, a branched cyclic glucan can be purified by the following procedure. After amylomaltase in a reaction solution is inactivated, a branched cyclic glucan and a cyclic α-1,4-glucan from the reaction solution are separated, for example, by gel filtration chromatography. Thereafter, the branched cyclic glucan can be obtained by using a conventional purifying method such as ethanol precipitation.

The enzyme of the present invention does not substantially catalyze a hydrolytic reaction, so that the yield of a cyclic glucan can be maintained at a high level for a long period of time in the cyclization reaction.

Characteristics of the cyclic glucan produced according to the above-mentioned (1) and (2), such as susceptibility to retrogradation, viscosity, and ability to prevent the retrogradation of starch, ability to form inclusion complex, digestibility, and energy conversion efficiency, can be rated by using any methods known to those skilled in the art. For example, methods described in Japanese Laid-open Publication No. 8-311103 can be used.

(Use of amylomaltase for improving food)

The amylomaltase of the present invention catalyzes the cyclization reaction of starch by acting on starch in food. As a result, the molecular size of starch in food is diminished and a cyclic glucan is produced. As described above, the cyclic glucan generated by this reaction has very low viscosity, high solubility, the ability to suppress the retrogradation of starch, and ability to include various substances. Thus, food can be improved by using the amylomaltase of the present invention. Typically, the amylomaltase is allowed to act on starch in food to accumulate a cyclic glucan, whereby physical properties of food (in particular, stability during storage) and mouthfeel can be improved.

Furthermore, the cyclic glucan generated by the cyclization reaction of starch has an $\alpha$-1,4-glucosidic bond and an $\alpha$-1,6-glucosidic bond. The cyclic glucan can be easily degraded into glucose by an enzyme present in an organism. Thus, the cyclic glucan has good digestibility and a high energy conversion efficiency.

The amylomaltase of the present invention has outstanding heat-resistance and does not substantially have hydrolytic activity, as described above. Therefore, in using amylomaltase for improving food, there is an advantage that it is possible to add an enzyme to a food material before or immediately after cooked by heating. Furthermore, there is an advantage that a reducing sugar having a low molecular weight is not accumulated to an unnecessarily degree.

The amylomaltase of the present invention can be used for improving a wide range of food (i.e., any food containing starch).

Examples of the food containing starch in a large amount include rice products (e.g., rice balls, sushi rice, lunch-box rice), Japanese desserts (e.g., bracken rice cakes, bean cakes, rice cakes, rice jellies, and rice bean cakes), snacks (e.g., Japanese crackers, rice crackers, potato chips, and other snacks), wheat products (e.g., breads, pies, pizzas, cakes, cookies, biscuits, and crackers), noodles (e.g., wheat noodles, buckwheat noodles, Chinese noodles, and pastas such as spaghetti and macaroni), gyoza skins, shumai skins, processed seafoods (e.g., fish sticks and fish-paste cakes), frozen or refrigerated processed foods, weaning foods, baby foods, pet foods, animal feeds, drinks (e.g., sports drinks), sports foods, and nutrient supplemental foods.

Rice products, Japanese desserts, snacks, wheat products and noodles, which contain starch in a large amount in particular, are examples of preferable foods to which the present invention is applied. Frozen or refrigerated processed foods in which the stability during storage at low temperature is important, are other examples of preferable foods.

The term "food material" as used herein refers to any material to produce the food described above, and any preparation provided during the process of producing the food.

Whether the food which is acted on by the amylomaltase of the present invention is improved can be confirmed by examining whether the molecular size of starch in the food has been diminished as a result of generation of a cyclic glucan. In the context of the present invention, it is defined that food is improved in a case where significant amount of a cyclic glucan is observed in food with the enzyme of the present invention added, compared with food with no enzyme added.

A food material, a food additive composition, and food improving agent respectively containing the amylomaltase of the present invention are useful in producing the improved food described above in the scope of the present invention. The amount of the enzyme to be added in these material and compositions can be appropriately selected by those skilled in the art.

Examples of the food additive composition includes condiments (e.g., soy sauces, soy sauce dips, Worcester sauces, broth bases, stew bases, curry bases, soup bases, mayonnaise, dressings, ketchup, and combined condiments).

Examples of the food improving agent includes anti-retrogradation agents and improving agents for steamed rice.

Examples

Hereinafter, the present invention will be described by way of illustrative examples. However, the present invention is not limited to these examples.

Example 1: Purification of amylomaltase from Thermus flavus ATCC 33923 strain cell extract

(1) Purification of amylomaltase

Thermus flavus ATCC 33923 strain was shake cultured in 33 liters of a medium (1% maltose, 0.4% yeast extract, O.8% polypeptone, 0.2% NaCl, pH 7.5) at 70°C for 18 hours. Cells were collected by centrifugation, and washed with 10 mM of $KH_2PO_4$-$Na_2HPO_4$ (pH 7.5) (hereinafter, referred to as "buffer A"). Thereafter, the cells were resuspended in the buffer A, and the cells were destroyed by sonication. The resulting cell debris was centrifuged to obtain a supernatant as a crude enzyme solution.

Ammonium acetate was added to the crude enzyme solution so as to adjust a final concentration of the salt to 1 mole (M). The resulting crude enzyme solution was allowed to stand overnight at 4°C, and loaded onto a Phenyl Toyopearl column (produced by Toso Inc.) equilibrated with the buffer A containing 1 M of ammonium sulfate. The column was washed with the buffer A containing 300 mM of ammonium sulfate, and an enzyme was eluted from the column by changing the concentration of ammonium sulfate in the buffer A from 300 mM to O mM, and active fractions were recovered and combined. The resulting enzyme solution was dialyzed against the buffer A.

The dialyzed enzyme solution was loaded onto a Source 15Q column (produced by Pharmacia Inc.) equilibrated with the buffer A. The column was washed with the buffer A. Then, the enzyme was eluted from the column by changing the concentration of NaCl in the buffer solution from 0 mM to 400 mM and active fractions were recovered and combined. The resulting enzyme solution was dialyzed against the buffer A.

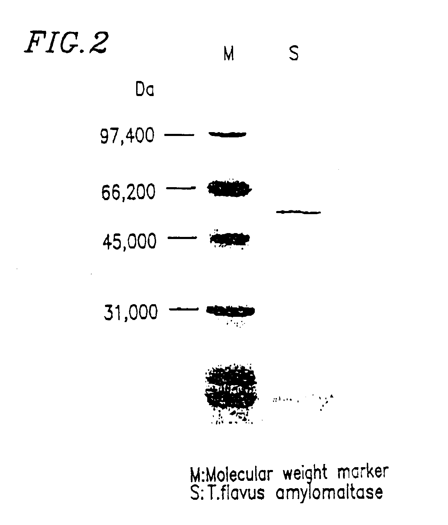

The dialyzed enzyme solution was loaded onto Superdex 75pg column (produced by Pharmacia Inc.) equilibrated with 50 mM of $KH_2PO_4$-$Na_2HPO_4$ (pH 7.5) containing 150 mM of NaCl. The enzyme was eluted from the column with the buffer A. The resulting enzyme solution was dialyzed against the buffer A to obtain a purified enzyme solution. The purified enzyme exhibited a single band by SDS polyacrylamide gel electrophoresis (Figure 2). The molecular weight of the amylomaltase measured by from the mobility in SDS-PAGE was 57,000.

(2) Activity measurement of amylomaltase

The activity of amylomaltase was measured by quantitating glucose generated when amylomaltase was allowed to act on 0.2% (w/v) maltotriose at 70°C for 10 minutes in 100 mM of a sodium acetate buffer (pH 5.5) (J. Biol. Chem. Vol. 268, pp. 1391-1396 (1993)).

The unit of activity was calculated based on the amount of enzyme which releases 1 $\mu$M of glucose per minute being defined as one unit.

Example 2: Various properties of amylomaltase derived from Thermus flavus ATCC 33923 strain

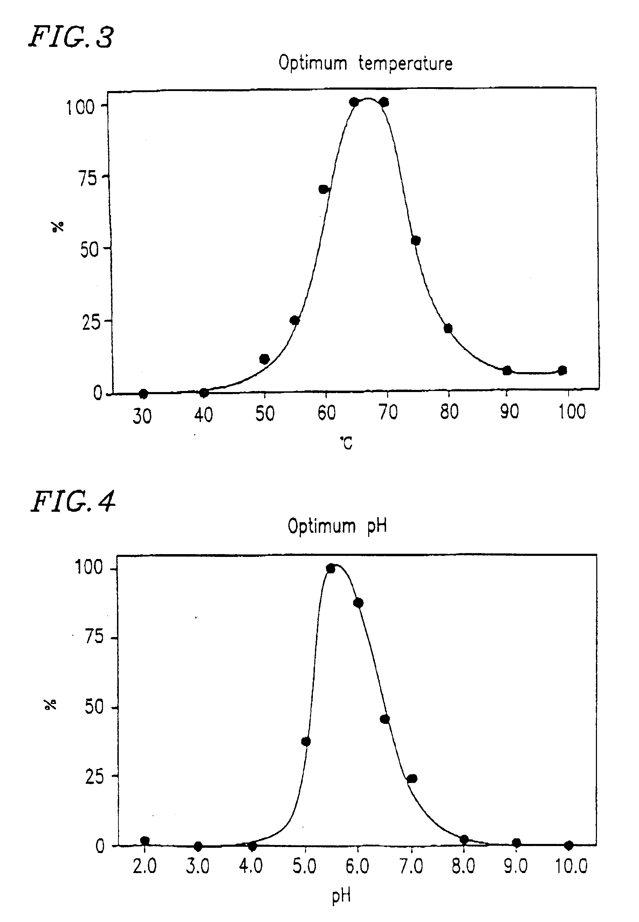

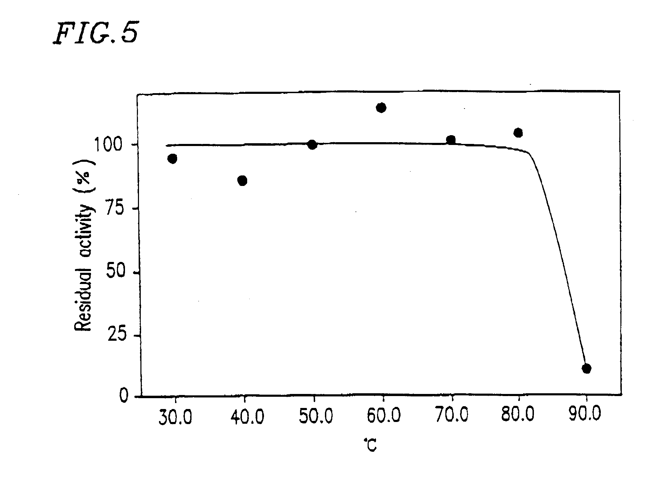

Enzymatic properties of the amylomaltase purified in Example 1 were analyzed using a method well-known to those skilled in the art. The reaction optimum temperature of the enzyme was 65°C to 70°C (Figure 3), and the reaction optimum pH was 5.5 (Figure 4). Furthermore, the enzyme retained almost 100% activity even after the heat treatment at 80°C for 10 minutes, and almost no inactivation was observed (Figure 5). However, the enzyme was completely inactivated at 100°C for 10 minutes (Figure 5).

Example 3: Action of amylomaltase derived from Thermus flavus ATCC 33923 strain on maltooligosaccharide

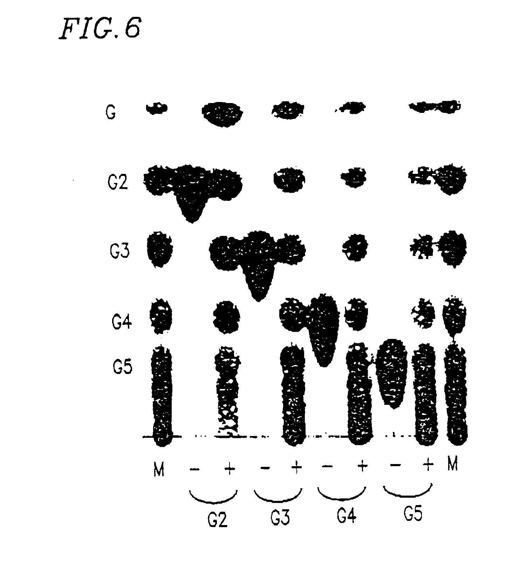

The action of the amylomaltase purified in Example 1 on maltooligosaccharide was examined. A portion of a purified enzyme was allowed to act on solutions containing maltooligosaccharides (maltose(G2), maltotriose(G3), maltoteraose (G4) and maltopentaose(G5)) in a final concentration of 1%. The reaction products were analyzed by thin layer chromatography (TLC). As shown in Figure 6, the amylomaltase of the present invention catalyzed a disproportionating reaction of these maltooligosaccharides, to generate maltooligosaccharide with various DPs as well as glucose(G). In Figutr 6, (-) lanes indicate starting maltooligosaccharide solutions, (+) lanes indicate reaction products, and M indicate a marker solution as a control.

Example 4: Determination of a partial amino acid sequence of amylomaltase derived from Thermus flavus ATCC 33923 strain

(1) Determination of an N-terminus amino acid sequence of purified amylomaltase

In the same way as in Example 1, an enzyme solution was obtained by chromatography using a Phenyl Toyopearl column. An enzyme solution was loaded onto a C4 column for HPLC (Vydac 214TP54 (0.46 x 25 cm), produced by Vydac) equibrated with an eluent which was 48.4% acetonitrile containing 0.1% trifluoroacetic acid (TFA). The enzyme was eluted from the column by changing the concentration of acetonitrile in the eluent from 48.4% to 52.4%. The resulting enzyme solution was concentrated *in vacuo.* An N-terminus amino acid sequence of the enzyme was determined by using a peptide sequencer. The sequence was found to be M-E-L-P-R-A-.

(2) Determination of an amino acid sequence of trypsin digested fragments from a purified amylomaltase

In the same way as in the above-mentioned (1), an enzyme was eluted from the C4 column for HPLC. The enzyme solution thus obtained was dried in vacuo, and then dissolved in a solution containing 8 M of urea, 0.4 M of $NH_4HCO_3$, 4.5 mM of dithiothreitol (DTT), and 10 mM of iodoacetoamide. Trypsin was added to the solution, and the mixture was allowed to react at 37°C for 24 hours. The solution containing the trypsin digested fragments thus obtained was loaded onto an ODS column for HPLC (Vydac 218TP54 (0.46 × 25 cm), produced by Vydac) equibrated with an eluent which was 1.6 % acetonitrile containing 0.06 % of TFA. The trypsin digested fragments were eluted from the column by changing the concentration of acetonitrile in the eluent from 1.6% to 78.4%. Three peak fractions respectively well separated from the other peaks were collected and concentrated *in vacuo.* Each N-terminus amino acid sequence of these peptide fragments was determined by the peptide sequencer. The sequences thus obtained were the following three kinds.

```
S-V-A-R-L-A-V-Y-P-V-Q-D-V-L-A-
M-N-Y-P-G-R-P-S-G-N-?-A-
I-I-G-D-M-P-I-F-V-A-E-D-
```

Example 5: Isolation of emylomaltase gene from Thermus flavus ATCC 33923 strain

(A) Construction of a genomic library of Thermus flavus ATCC 33923 strain

(A1) Preparation of genomic DNA

The strain was shake-cultured at about 70°C for 10 to 16 hours in a 2-liter of shaking flask in which 1000 ml of a medium containing 0.89% of trypton, 0.4% of yeast extract, and 0.2% sodium chloride was placed. Cells were collected by centrifugation, and genomic DNA was prepared by a phenol method (Saito and Miura, Biochimica et Biophysica Acta, 72, 619 (1963)).

(A2) Insertion of genomic DNA into λ-ZAP expression vector

The genomic DNA obtained in (A1) was partially digested with a restriction enzyme Sau3AI. Thereafter, the genomic DNA was subjected to sodium chloride density gradient centrifugation, whereby fractions having DNA fragments of 5 to 10 Kbp were obtained. The DNA fragments thus obtained were linked to BamHI treated λ-ZAP expression vector (produced by Stratagene) by using T4DNA ligase. This reaction solution was treated using LAMBDA INN (Nippon Gene Co., Ltd) which is an *in vitro* packaging kit, whereby a recombinant λ phage suspension was obtained.

(A3) Introduction of recombinant λ phage into *E. coli*

*E. coli* VCS257 strain was shake-cultured in an L medium (10 gram per liter (g/l) of trypton (Difco), 5 g/l yeast extract (Difco), 10 g/l of NaCl, pH 7.0) containing 10 mM of magnesium chloride, and suspended in 20 ml of 10 mM magnesium chloride solution. Then, the suspension was mixed with the recombinant λ phage, and the mixture was kept at 37°C for 20 minutes. Thereafter, the mixture was seeded on the plate where an L top agar medium (10 g/l trypton (Difco), 5 g/l of yeast extract (Difco), 10 g/l of NaCl, pH 7.0, 0.8% (w/v) Agar) was overlayed on an L agar medium (10 g/l of tryptone (Difco), 5 g/l of yeast extract (Difco), 10 g/l of NaCl, pH 7.0, 1.5% (w/v) Agar). The medium thus obtained was incubated overnight at 37°C to obtain some plates on which plaques were observed.

(B) Screening of a genomic library of Thermus flavus ATCC 33923 strain using an oligonucleotide probe

(B1) Preparation of an oligonucleotide probe for isolating an amylomaltase gene

Synthetic oligo DNA (5'-GCIGTITAYCCIGTICARGAYGT-3' (the description of a nucleic acid is in accordance with the IUPAC nucleic acid code) corresponding to a partial amino acid sequence (A-V-Y-P-V-Q-D-V) of the amylomaltase from Thermus flavus ATCC 33923 strain, which was determined in Example 4, was prepared. The synthetic oligo DNA was radioactively labeled, and used as a probe for isolating an amylomaltase gene.

(B2) Selection of recombinant λ phage containing an amylomaltase gene

The phage plaques were transferred to nylon filters (manufactured by Amersham Corp.) by placing the filters in contact with the plate prepared in (A3). Phage DNAs on the filters were denatured using an alkali solution and then neutralized using a neutralizing solution. The recombinant λ phage DNAs were immobilized onto the filters by baking. These filters were subjected to hybridization reaction by using the probe produced in (B1). Recombinant λ phage containing an amylomaltase gene was selected by detecting a positive signal on an autoradiogram.

(B3) Sub-cloning of recombinant λ phage containing an amylomaltase gene to a plasmid vector

The recombinant λ phage obtained in (B2), E. coli (XLI-Blue MRF strain), a helper phage (ExAssist) were mixed, and the mixture was kept at 37°C for 15 minutes. Thereafter, 5 mL of the L medium was added to the mixture, and the resulting mixture was shake-cultured at 37°C for 4 to 6 hours. This culture broth was heat-treated at 70°C for 20 minutes and centrifuged to remove a supernatant. Thus, a phage solution was obtained. A mixture of 20 μl of the phage solution and 200 μl of E. coli (XLOLR strain) was prepared, and kept at 37°C for 15 minutes. Then, a part or the entire amount of the mixture was plated on the L agar medium containing 50 μg/μl of kanamycin, and the medium was kept at 37°C for 10 to 18 hours. Plasmid DNA was extracted from generated colonies by an alkali SDS method (Sambrook et al., Molecular Cloning, supra). Thus, plasmid DNA containing an amylomaltase gene was obtained.

(C) Determination of a nucleotide sequence of amylomaltase gene derived from Thermus flavus ATCC 33923 strain

The plasmid DNA obtained in the above (B3) was determined for its nucleotide sequence by using a DNA sequencer (manufactured by ABI). The entire nucleotide sequence of the amylomaltase gene is represented in SEQ. ID NO. 2 of Sequence Listing. SEQ.ID NO. 1 of Sequence Listing is a corresponding deduced amino acid sequence.

Example 6: Recombinant production of heat-resistant amylomaltase)

(A) Amplification of an amylomaltase gene by PCR

PCR (25 cycles (at 98°C for 10 seconds and at 68°C for 1 minute)) was performed under the condition containing a final concentration of 10% of glycerol, using two kinds of oligonucleotides (P1: 5'-TTTCATATGGAGCTTCCCCGCGCTTTCGGTCTGCTT-3', P2: 5'-TTTGAATTCGGGCTGGTCCACCTAGAGCCGTTCCGT) as primers, and the genomic DNA or plasmid obtained from Thermus flavus ATCC 33923 strain in Example 5 or a plasmid as a template. By this amplification, an amylomaltase gene in which a restriction enzyme NdeI site is added at the N-terminus of a structural gene and a restriction enzyme EcoRI site is added at the C-terminus thereof was obtained. Amplified DNA was completely digested with the restriction enzymes NdeI and EcoRI, and then, subjected to agarose electrophoresis, whereby DNA fragment of about 1.5 kb containing an amylomaltase gene was recovered.

(B) Construction of an expression vector

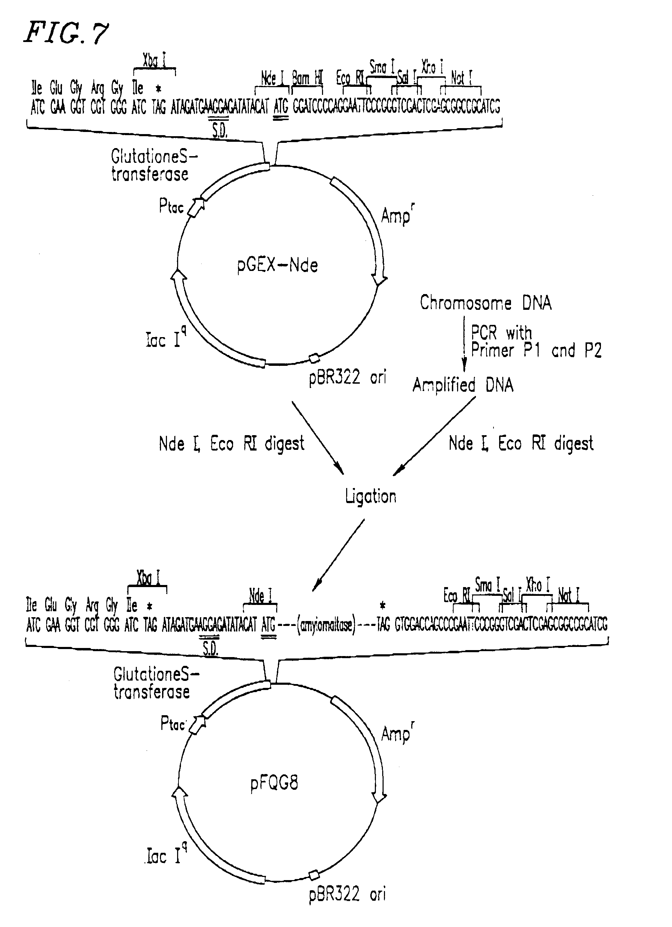

An oligonucleotide adaptor having the following sequence was inserted into a BamHI site of commercially available E. coli expression vector pGEX-5X-3 (produced by Pharmacia Inc.) to obtain a plasmid pGEX-Nde (see Figure 7).

**5'-GATCTAGATAGATGAAGGAGATATACATATGG**

**ATCTATCTACTTCCTCTATATGTATACCCTAG-5'**

The about 1.5 kb DNA fragment containing an amylomaltase gene prepared in (A) was introduced into the NdeI-EcoRI site of an expression plasmid pGEX-Nde to obtain an amylomaltase expression plasmid pFQG8 (see Figure 7).

(C) Expression of heat-resistant amylomaltase in E. coli

E. Coli TG-1 strain transformed with the amylomaltase expression plasmid pFQG8 was cultured in 1 liter of an LB medium (1% of trypton, 0.5% yeast extract, 1% NaCl, pH 7.5) containing ampicillin in a final concentration of 100 μg/ml at 37°C until the late logarithmic growth phase (about 6 hours). Thereafter, IPTG in a final concentration of about 0.1 mM was added to the culture medium. After the culture was further continued at 37°C for 16 hours, the medium was centrifuged to collect cells. The cells thus obtained were washed twice with 300 ml of the buffer A, and then dispersed in 60 ml of the buffer A. The cells were destroyed by sonication, and a supernatant was obtained by centrifugation as a crude enzyme solution. The amylomaltase activity of the crude enzyme solution thus obtained was 30 units/ml, which was about 500 times (per 1 ml of culture broth) the activity obtained when Thermus flavus ATCC 33923 strain was cultured.

Example 7: Production of a cyclic glucan

(A) Production of a cyclic α-1,4-glucan (Production of cycloamylose using the amylomaltase of the present invention)

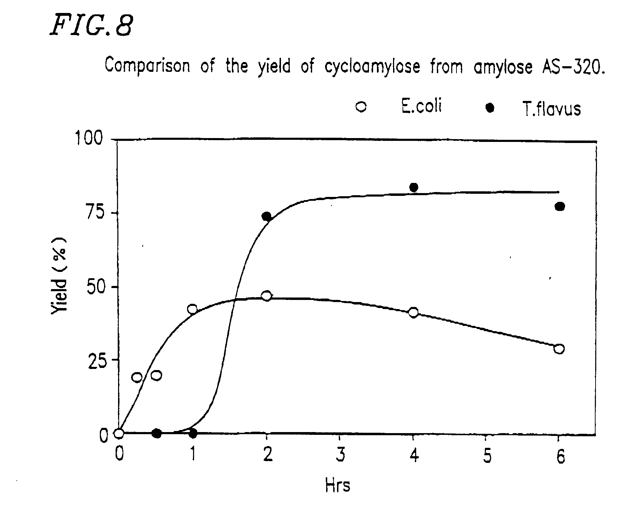

In 1 ml of a buffer (100 mM of sodium acetate buffer (pH 5.5) including 9% (v/v) DMSO) containing enzymatically synthesized amylose AS-320 (produced by Nakano vinegar Co., Ltd.) in a final concentration of 0.2%, 0.07 units of the purified amylomaltase obtained in Example 1 was allowed to act at 70°C. An unreacted linear amylose was degraded into glucose by using glucoamylase, and then, cycloamylose in the reaction product was quantitated. The enzyme of the present invention cyclized amylose, and cycloamylose almost linearly increased until, after 2 hours of reaction, finally reaching a yield of about 80% (weight ratio with respect to amylose used as substrate) (Figure 8). For comparison, amylomaltase derived from E. coli was allowed to act under the same conditions except that the reaction was performed at 30°C. In this case, the maximum yield was as low as about 50%, and the yield decreased with the passage of reaction. This is presumably because E. coli amylomaltase degraded the produced cycloamylose due to its weak hydrolytic activity, while catalyzing the cyclization reaction. Regarding the amylomaltase of the present invention, no decrease in yield of cycloamylose was observed even after 6 hours of reaction. Thus, it is apparent that the amylomaltase of the present invention does not substantially catalyze the hydrolytic reaction, and hence is very suitable as an enzyme for producing cycloamylose.

A sample after 6 hours of reaction was digested by glucoamylase to degrade unreacted linear amylose into glucose. Thereafter, cycloamylose was purified by precipitation with ethanol. The cycloamylose thus obtained was analyzed by a carbohydrate analysis system (eluting system: DX300, detector: PAD-2, analysis column: CarboPacPA100) manufactured by DIONEX. Elution was performed under the conditions of a flow rate: 1 ml/min., concentration of NaOH: 150 mM, and concentration of sodium acetate: 0 min.- 50 mM, 2 min.-50 mM, 37 min.-350 mM (Gradient curve No. 3), 45 min.-850 mM (Gradient curve No. 7), 47 min.-850 mM.

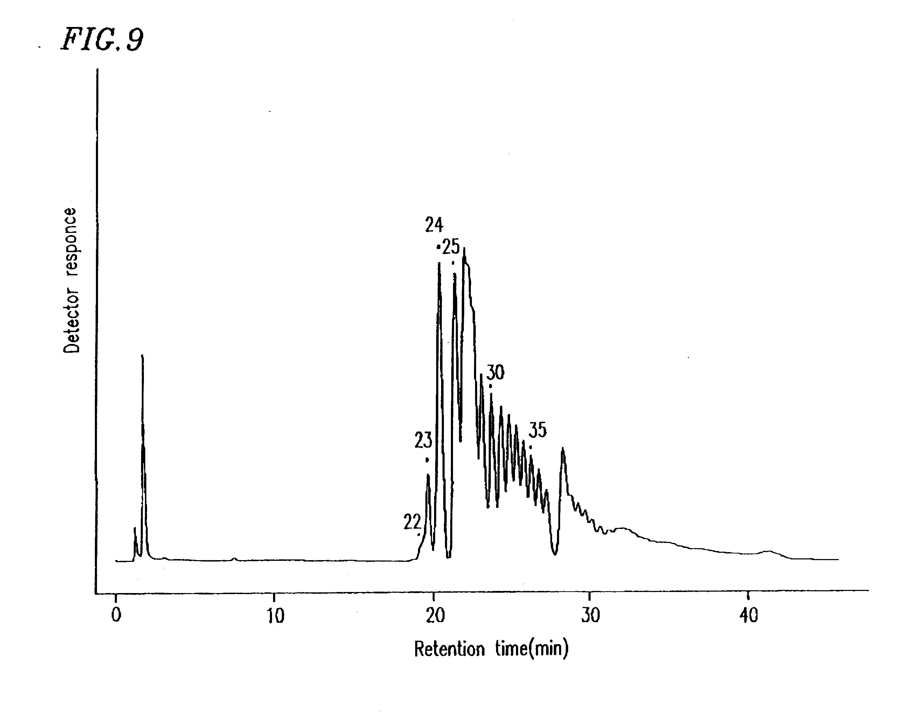

As shown in Figure 9, the purified cycloamylose was a mixture having a DP of 21 or more. The DP of cycloamylose was determined by the elusion position of cycloamylose standard having a DP of 23 to 26 (Takaha et al., J. Biol.Chem. Vol. 271, pp. 2902-2908 (1996)).

(B) Production of a branched cyclic glucan

(B1) Production of a branched cyclic glucan using the amylomaltase of the present invention

In 400 ml of 10 mM citrate buffer (pH 7.0), 2 grams of waxy corn starch (Sanwa cornstarch Co., Ltd.) was dissolved by heating. Thereafter, 10 units of the enzyme obtained in Example 6 was added to the mixture, and the resulting mixture was allowed to react at 70°C for 6 hours.

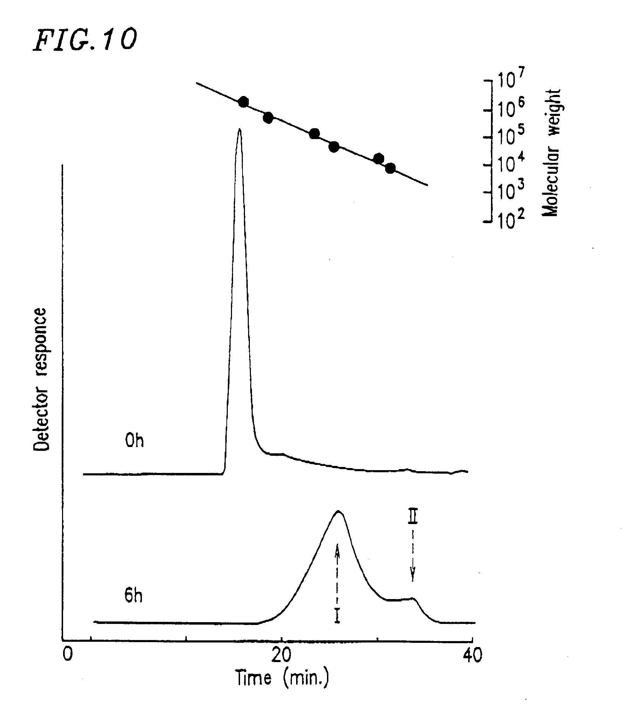

The reaction solution was heated at 100°C for 10 minutes, whereby the enzyme was inactivated. Then, the resulting reaction solution was subjected to centrifugation, whereby denatured protein was removed. A ten-fold volume of ethanol was added to the supernatant to precipitate the glucan. Then, the precipitant thus obtained was lyophilized to obtain 1.8 g of powders which were later confirmed to contain the cyclic glucans. The powders were analyzed by gel filtration chromatography. The powdery precipitation (20 mg) was dissolved in 1 ml of 100 mM NaCl solution, and 250 μl of it was loaded onto a connected column of Superose 6 (φ1 cm × 30 cm, Pharmacia, Inc.) and Superdex 30 (φ1 cm × 30 cm, Pharmacia, Inc.), and eluted with a 100 mM NaCl solution. As shown in Figure 10, the amylopectin (Oh) was eluted in the void volume under this elution condition. On the other hand, the product whose molecular size has been diminished by the reaction with amylomaltase (6h) exhibited two fractions, i.e., Peak I having an average molecular weight of about 30,000 and Peak II having an average molecular weight of about 3000. Peak II was found to mainly correspond to a cyclic glucan having only α-1,4-glucosidic bonds. On the other hand, Peak I was found to correspond to a branched cyclic glucan having α-1,6-glucosidic bonds. This chromatography procedure was repeated four times, and Peak I was respectively fractionated. The branched cyclic glucan was precipitated from the combined fractions by the addition of a 10-fold volume of ethanol. The precipitant was centrifuged, recovered, and thereafter lyophilized to obtain 15 mg of the branched cyclic glucan. The above-mentioned molecular weights of the cyclic glucans were measured using enzymatically synthesized amylose (Nakano vinegar Co., Ltd.) as a standard.

(B2) Quantification of the α-1,6-bonds in the glucan in Peak I

It was proved that the glucan in Peak I obtained in (B1) has an α-1,6-bond by the method stated below.

An α-1,6-bond in a glucan is hydrolysed selectively by a debranching enzyme (EC. 3.2.1.68 and EC. 3.2.1.41). By determining an increase in reducing power, the α-1,6-bonds can be quantified in the glucan. This is a conventional method in the art.

In 1 ml of 10 mM sodium acetate (PH 5.5), 1 mg of the Peak I fraction obtained in (B1) was dissolved and was reacted with a sufficient amount of the debranching enzyme. After the reaction was stopped by heating, the increase in reducing power and the entire amount of sugars were determined. As a result, it was shown that the glucan in Peak I obtained in (B1) had about 7% of α-1,6-bonds.

(B3) Quantification of the glucan

It was found that the glucan in Peak I obtained in (B1) has a cyclic structure, and the cyclic structure portion of the glucan was further analyzed, by the method stated below.

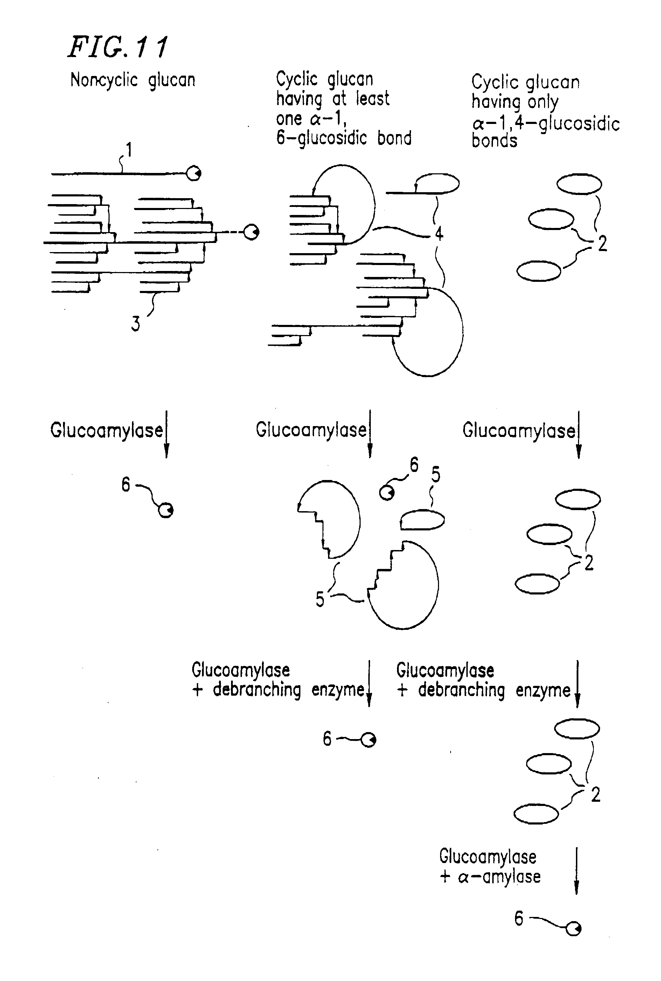

Glucoamylase is an enzyme capable of hydrolyzing successively the α-1,4-glucosidic bonds from the non-reducing end of a glucan such as starch. It is known that the enzyme also hydrolyzes the α-1,6-glucosidic bond from the non-reducing end, although the hydrolysis proceeds more slowly. As shown in Figure 11, amylose (1) and amylopectin (3), which do not have a cyclic structure are degraded completely to glucose (6) by glucoamylase. However, in the case of glucans (4) and (2) having in their molecule a cyclic structure, only non-cyclic structure portions of the glucans are degraded by glucoamylase, and their cyclic structure portions remain as a material which is not subject to the degradation by glucoamylase (hereinafter referred to as a glucoamylase resistant component). Moreover, the glucoamylase resistant component is classified into an inner branched cyclic glucan (5) and a cyclic α-1,4-glucan (2) according to their sensitivities to a debranching enzyme. That is, the glucoamylase resistant component which is not degraded by a combination of the debranching enzyme and glucoamylase, is understood to be the cyclic α-1,4-glucan (2). The cyclic α-1,4-glucan (2) can be completely degraded to glucose by the combined use of an end-type α-amylase and glucoamylase. On the other hand, the glucoamylase resistant component, which is degraded by the combined use of the debranching enzyme and glucoamylase, is understood to be the inner branched cyclic glucan (5).

By using the above-mentioned method, the branched cyclic glucan (fractions of Peak I) obtained in (B1) and amylopectin as a control were analyzed for the presence of a cyclic structure.

After dissolving 10 mg of the branched cyclic glucan obtained in (B1) and 10 mg of amylopectin in l ml of DMSO, respectively, each solution was immediately diluted with 8 ml of 100 mM sodium acetate buffer. The diluted solution was dispensed in aliquots (900 μl) into four tubes. Then, 100 μl each of (i) distilled water, (ii) a glucoamylase solution, (iii) a mixture solution of a debranching enzyme and glucoamylase, and (iv) a mixture solution of an end-type α-amylase and glucoamylase was added to each tube. Each of the resulting mixtures was allowed to react at 40°C for 4 hours. After the reaction was terminated, the glucose produced was measured by a commercially available glucose quantitation kit (manufactured by VIAKO PURE CHEMICAL INDUSTRIES, LTD.). The percentages of the non-cyclic structure portion, the cyclic structure portion having only α-1,4-bond, and the cyclic structure portion having α-1,6-bond in the sample glucan were determined according to the following calculation formulae.

$$\text{Non-cyclic structure portion (\%)} = \frac{x - c}{z - c} \times 100$$

$$\text{Cyclic structure portion (\%) having an } \alpha\text{-1,6-bond} = \frac{y - x}{z - c} \times 100$$

$$\text{Cyclic structure portion (\%) having only } \alpha\text{-1,4-bonds} = \frac{z - y}{z - c} \times 100$$

where c, x, y and z represent the amount of glucose produced in the reaction solution of (i), (ii), (iii) and (iv), respectively. Table I shows the results.

Table 1

|  | Amylopectin (%) | Branched glucan obtained in (B1) |
|---|---|---|
| Non-cyclic structure portion | 100.0 | 89.3 |
| Cyclic structure portion having only $\alpha$-1,4-bonds | 0.0 | 0.1 |
| Cyclic structure portion having an $\alpha$-1,6-bond | 0.0 | 10.6 |

It can be seen from the results that the branched glucan contains an inner branched cyclic glucan having at least one $\alpha$-1,6-glucosidic bond in the cyclic structure portion.

Example 8: Application of the amylomaltase of the present invention to food

First, 90 g of commercially available powders for bracken rice cake (main components: starch of sweet potato, produced by Yamamoto koshi shouten) and 360 ml of water were thoroughly mixed in a pan. The pan was heated while stirring the mixture. The pan was removed from heat when the bracken rice cake powders were made into transparent like a rice cake. The stirring was further continued. When the temperature of the pan was decreased to about 80°C, 200 units of the enzyme obtained in Example 6 were added to the pan. Thereafter, the mixture was continued to be stirred at about 70°C for 15 minutes. The rice cake thus obtained was poured into a flat container, and the container was cooled in a refrigerator, whereby a bracken rice cake ("warabi-mochi" in Japanese) was produced. As a control, a bracken rice cake without an enzyme added was similarly produced.

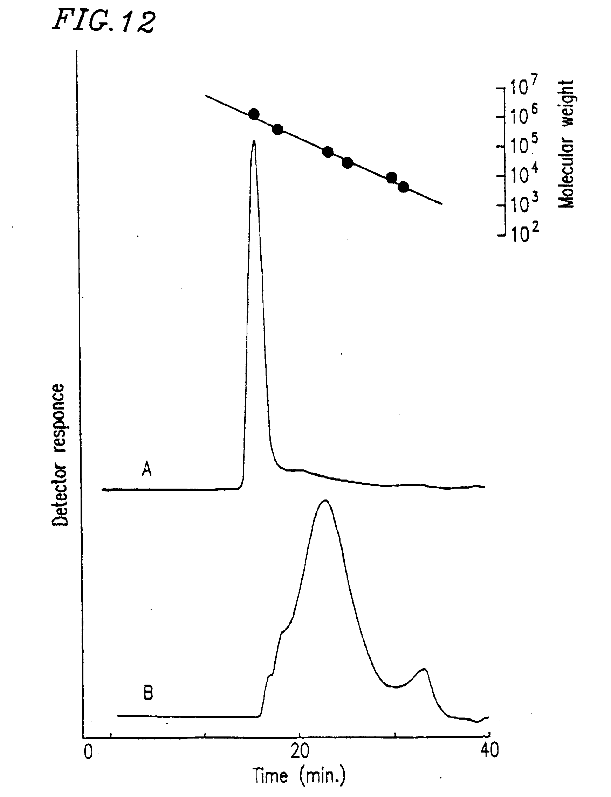

An 1 gram portion of each of the obtained bracken rice cakes was dissolved in 20 ml of hot water to extract starch. The extracted starch was analyzed by gel permeation chromatography, using the method similar to that described in (B1) in Example 7. Figure 12 shows the results. It is shown in this figure that in the case of the starch from the bracken rice cake with the enzyme added, the molecular size has been diminished as a result of a cyclization reaction.

Furthermore, measurements similar to those described in Example 7 were performed, whereby it was confirmed that the glucan diminished in its molecular size has cyclic structure.

The enzyme of the present invention is a class of amylomaltase having particularly outstanding properties for producing a cyclic glucan and for improving food among the enzymes found so far. As described in the above-mentioned examples, the enzyme of the present invention has a reaction optimum temperature of 65° to 70°C, and can be used even at 80°C. Furthermore, the enzyme of the present invention can easily be inactivated by being heated at 100°C for 15 minutes after the cyclization of an $\alpha$-glucan, and hence, can prevent a reverse reaction. Accordingly, in producing a cyclic glucan, the previous problems involving the contamination of microorganisms and the retrogradation of an $\alpha$-glucan caused by a low reaction temperature, the hydrolysis of a product, and a reverse reaction can be overcome, and a cyclic glucan can be produced efficiently. Furthermore, in improving food, the molecular size of starch in food can be diminished by adding an enzyme to the food material before or immediately after cooking by heating, whereby a cyclic glucan can be generated.

Various other modifications will be apparent to and can be readily made by those skilled in the art without departing from the scope and spirit of this invention. Accordingly, it is not intended that the scope of the claims appended hereto be limited to the description as set forth herein, but rather that the claims be broadly construed.

## Claims

1. Amylomaltase having the following properties:

    1) function: generating a cyclic glucan by an intramolecular transglycosylation reaction, using an $\alpha$-glucan as a substrate;
    2) reaction specificity: not substantially catalyzing a hydrolytic reaction;
    3) reaction optimum temperature: 65°C to 70°C;
    4) heat-resistance: maintaining activity at 60°C for at least 10 minutes and being inactivated at 100°C in 15 minutes: and
    5) reaction optimum pH: pH 5.5.

2. Amylomaltase according to claim 1, which is derived from Thermus flavus ATCC 33923.

3. Amylomaltase which is a) or b) stated below:

    a) amylomaltase having the amino acid sequence from Met at 1st position to Leu at 500th position represented in SEQ.ID NO. 1 of Sequence Listing; or

b) amylomaltase with heat-resistance, having an amino acid sequence in which one or more amino acids of a) are subjected to deletion, substitution, or addition.

4. A gene encoding amylomaltase which is a) or b) stated below:

a) amylomaltase having the amino acid sequence from Met at 1st position to Leu at 500th position represented in SEQ.ID NO. 1 of Sequence Listing; or
b) amylomaltase with heat-resistance, having an amino acid sequence in which one or more amino acids of a) are subjected to deletion, substitution, or addition.

5. An amylomaltase gene having DNA which is a) or b) stated below:

a) DNA having a nucleotide sequence represented in SEQ.ID NO. 2 of Sequence Listing; or
b) DNA which hybridizes to DNA of a) under stringent conditions, and encodes a heat-resistant protein having amylomaltase activity.

6. An expression vector containing the heat-resistant amylomaltase gene of claim 4.

7. An expression vector containing the heat-resistant amylomaltase gene of claim 5.

8. A microorganism transformed with the expression vector of claim 6.

9. A microorganism transformed with the expression vector of claim 7.

10. A method for producing amylomaltase, comprising the steps of:

culturing the transformed microorganism of claim 8; and
collecting and purifying amylomaltase produced by the culture.

11. A method for producing amylomaltase, comprising the steps of:

culturing the transformed microorganism of claim 9; and
collecting and purifying amylomaltase produced by the culture.

12. Amylomaltase produced by the method of claim 10.

13. Amylomaltase produced by the method of claim 11.

14. A method for producing a cyclic glucan, comprising the steps of:

cyclizing an $\alpha$-glucan using the amylomaltase of claim 1; and
collecting and purifying the cyclic glucan.

15. A method according to claim 14, wherein the cyclic glucan includes a cyclic $\alpha$-1,4-glucan.

16. A method according to claim 14, wherein the cyclic glucan includes a branched cyclic glucan.

17. A method according to claim 14, wherein a branching enzyme is further used in the step of cyclization.

18. A method for producing food, comprising the step of adding the amylomaltase of claim 1 to a food material before or immediately after cooking by heating, wherein the amylomaltase acts on a starch in the food material to produce a cyclic glucan.

19. A method according to claim 18, wherein the food is selected from the group consisting of rice products, Japanese desserts, snacks, wheat products, noodles, gyoza skins, shumai skins, processed seafoods, frozen or refrigerated processed foods, weaning foods, baby foods, pet foods, animal feeds, drinks, sports foods, and nutrient supplemental foods.

**20.** A food material, a food additive composition, and a food improving agent, containing the amylomaltase of claim 1.

## FIG.1

Amylose

Amylomaltase

Cycloamylose

Inactivation of amylomaltase

Glucoamylase

Glucose

Removal of glucose

Purified cycloamylose

Cyclization step

Amylomaltase inactivation step

Glucoamylase treatment step

Glucose removal step

FIG.2

M:Molecular weight marker
S:T.flavus amylomaltase

*FIG.3*

Optimum temperature

*FIG.4*

Optimum pH

*FIG.5*

# FIG.6

*FIG.7*

# FIG.8

Comparison of the yield of cycloamylose from amylose AS-320.

○  E.coli      ●  T.flavus

FIG.9

FIG.10

# FIG.11

**Noncyclic glucan**

**Cyclic glucan having at least one α-1, 6-glucosidic bond**

**Cyclic glucan having only α-1,4-glucosidic bonds**

Glucoamylase

Glucoamylase

Glucoamylase

Glucoamylase + debranching enzyme

Glucoamylase + debranching enzyme

Glucoamylase + α-amylase

# FIG.12

**Annex to the description**

SEQUENCE LISTING

(1) GENERAL INFORMATION

(i) APPLICANT: Ezaki Glico Co., Ltd.

(ii) TITLE OF INVENTION: HEAT-RESISTANT AMYLOMALTASE

(iii) NUMBER OF SEQUENCES: 2

(iv) CORRESPONDENCE ADDRESS:
    (A) ADDRESS: Ezaki Glico Co., Ltd.
    (B) STREET: 4-6-5, Utajima, Nishiyodogawa-ku
    (C) CITY: Osaka-shi
    (D) STATE: Osaka-hu
    (E) COUNTRY: Japan
    (F) ZIP: 555-8502

(v) COMPUTER READABLE FORM:
    (1) MEDIUM TYPE: Diskette. 3.50 inch. 1.4 MB format
    (2) COMPUTER: EPSON
    (3) OPERATING SYSTEM: MS-DOS ver.2.11
    (4) SOFTWARE: Word Perfect (ASCII file)

(Vii) PRIOR APPLICATION DATA:
    (A) APPLICATION NUMBER: (1) JP 9-122635
                               (2) JP 10-125121
    (B) FILING DATE: (1) 13-March-1997
                   (2) 7-May-1998

(2) INFORMATION FOR SEQ ID NO. 1
(1) SEQUENCE CHARACTERISTICS:
    (A) LENGTH: 500

(B) TYPE: amino acid

(D) TOPOLOGY: linear

(ii) MOLECULAR TYPE: protein

(Xi) SEQUENCE DESCRIPTION: SEQ ID NO. 1:

Met Glu Leu Pro Arg Ala Phe Gly Leu Leu Leu His Pro Thr Ser Leu
                5                    10                    15

Pro Gly Pro Tyr Gly Val Gly Val Leu Gly Arg Glu Ala Arg Asp Phe
                20                   25                   30

Leu Arg Phe Leu Lys Glu Ala Gly Gly Arg Tyr Trp Gln Val Leu Pro
            35                   40                   45

Leu Gly Pro Thr Gly Tyr Gly Asp Ser Pro Tyr Gln Ser Phe Ser Ala
            50                   55                   60

Phe Ala Gly Asn Pro Tyr Leu Ile Asp Leu Arg Pro Leu Ala Glu Arg
65                   70                   75                   80

Gly Tyr Val Arg Leu Glu Asp Pro Gly Phe Pro Gln Gly Arg Val Asp
                85                   90                   95

Tyr Gly Leu Leu Tyr Ala Trp Lys Trp Pro Ala Leu Lys Glu Ala Phe
                100                  105                  110

Arg Gly Phe Lys Glu Lys Ala Ser Pro Glu Glu Arg Glu Ala Phe Ala
            115                  120                  125

Ala Phe Arg Glu Arg Glu Ala Trp Trp Leu Glu Asp Tyr Ala Leu Phe
            130                  135                  140

Met Ala Leu Lys Gly Ala His Gly Gly Leu Pro Trp Asn Arg Trp Pro
145                  150                  155                  160

Leu Pro Leu Arg Lys Arg Glu Glu Lys Ala Leu Arg Glu Ala Lys Ser
                165                  170                  175

Ala Leu Ala Glu Glu Val Ala Phe His Ala Phe Thr Gln Trp Leu Phe
            180                  185                  190

Phe Arg Gln Trp Gly Ala Leu Lys Ala Glu Ala Glu Ala Leu Gly Ile
            195                  200                  205

Arg Ile Ile Gly Asp Met Pro Ile Phe Val Ala Glu Asp Ser Ala Glu
210                  215                  220

Val Trp Ala His Pro Glu Trp Phe His Leu Asp Glu Glu Gly Arg Pro
225                  230                  235                  240

Thr Val Val Ala Gly Val Pro Pro Asp Tyr Phe Ser Glu Thr Gly Gln
                245                  250                  255

Arg Trp Gly Asn Pro Leu Tyr Arg Trp Asp Val Leu Glu Arg Glu Gly
            260                  265                  270

Phe Ser Phe Trp Ile Arg Arg Leu Glu Lys Ala Leu Glu Leu Phe His
275                     280                 285

Leu Val Arg Ile Asp His Phe Arg Gly Phe Glu Ala Tyr Trp Glu Ile
290                     295                 300

Pro Ala Ser Cys Pro Thr Ala Val Glu Gly Arg Trp Val Lys Ala Pro
305                 310                 315                 320

Gly Glu Lys Leu Phe Gln Lys Ile Gln Glu Val Phe Gly Glu Val Pro
325                     330                 335

Val Leu Ala Glu Asp Leu Gly Val Ile Thr Pro Glu Val Glu Ala Leu
340                     345                 350

Arg Asp Arg Phe Gly Leu Pro Gly Met Lys Val Leu Gln Phe Ala Phe
355                     360                 365

Asp Asp Gly Met Glu Asn Pro Phe Leu Pro His Asn Tyr Pro Ala His
370                     375                 380

Gly Arg Val Val Val Tyr Thr Gly Thr His Asp Asn Asp Thr Thr Leu
385                 390                 395                 400

Gly Trp Tyr Arg Thr Ala Thr Pro His Glu Lys Ala Phe Met Ala Arg
405                 410                 415

Tyr Leu Ala Asp Trp Gly Ile Thr Phe Arg Glu Glu Glu Val Pro
420                     425                 430

Trp Ala Leu Met His Leu Gly Met Lys Ser Val Ala Arg Leu Ala Val
435                     440                 445

Tyr Pro Val Gln Asp Val Leu Ala Leu Gly Ser Glu Ala Arg Met Asn
450                     455                 460

Tyr Pro Gly Arg Pro Ser Gly Asn Trp Ala Trp Arg Leu Leu Pro Gly
465                 470                 475                 480

Glu Leu Ser Pro Glu His Gly Ala Arg Leu Arg Ala Met Ala Glu Ala
485                     490                 495

Thr Glu Arg Leu
500

(2) INFORMATION FOR SEQ ID NO. 2
(i) SEQUENCE CHARACTERISTICS:
    (A) LENGTH: 1503
    (B) TYPE: nucleic acid
    (C) STRANDENESS: double
    (D) TOPOLOGY: linear
(ii) MOLECULAR TYPE: Genomic DNA
(vi) ORIGINAL SOURCE

```
ORGANISM: Thermus flavus
STRAIN: ATCC 33923
(Xi) SEQUENCE DESCRIPTION: SEQ ID NO. 2:
ATGGAGCTTC CCCGCGCTTT CGGTCTGCTT CTCCACCCCA CGAGCCTCCC CGGCCCCTAC    60
GCCGTCGGCG TCCTGGGCCG GGAGGCCCGG GACTTCCTCC GCTTCCTCAA GGAGGCCGGG   120
GGCCGGTACT GGCAGGTCCT CCCCTTGGGC CCCACGGCCT ATGGCGACTC CCCCTACCAG   180
TCCTTCAGCG CCTTCGCCGG AAACCCCTAC CTCATAGACC TGAGGCCCCT CGCCGGAAAGG  240
GGCTACGTGC GCCTGGAGGA CCCCGGCTTC CCCCAAGGCC GGGTCGACTA CGGCCTCCTC   300
TACGCCTGGA AGTCGCCCGC CCTGAAGGAG GCCTTCCCGG GCTTCAAGGA AAAGGCCTCC   360
CCCGAGGAGC GGGAGGCCTT CGCCGCCTTC CGGGAGAGCG AGGCCTCGGTG GCTCGAGGAC  420
TACGCCCTCT TCATGGCCCT GAAGGGGGCG CACGGGGGCC TTCCCTCGAA CCGGTCGGCCC  480
CTTCCCCTGC GGAAGCGGGA AGAGAAGGCC CTTAGGCGAGG CGAAAAGCCC CTTGGCCGAG   540
GAGGTCGCCT TCCACGCCTT CACCCAGTCG CTCTTCTTCC GCCAGTCGGGC GGCCTTGAAG   600
GCCGAGGCCG AGGCGTTCGG CATCCCGGATC ATCGGGGACA TGCCCATCTT CGTCGCCCGAG  660
GACTCCGCCG AGGTCTCGGC CCACCCCGAG TCGTTTCACC TCGACGAGGA GGGCCCGCCCC   720
ACGGTCGGTGG CCGCCCGTCCC CCCCGACTAC TTCTCGGAGA CCGGCCAGCG CTGCGGGTAAC  780
CCCCTTTACC GCTCGGACCGT TTTGGACCGG GAGCCGTTCT CCTTCTGGAT CCGCCCGTCTG   840
GAGAAGGCCC TCGAGCTCTT CCACCTGGTG CCGCATAGACC ACTTCCCGCGG CTTTGAGGCC   900
TACTCGGGAGA TCCCCGCAAG CTGCCCCACG CGCGGTCGGAGG GCCGCTCGGGT CAAGCCCCCG   960
GGGCAGAAGC TCTTCCAGAA GATCCAGGAG GTCTTCGGCG AGGTCCCCCGT CCTCGCCGAG  1020
GACCTCGGGG TCATCACCCC CGAGGTCGGAG GCCCTGCCGCG ACCGGCTTCGG CCTTCCCGGG  1080
ATGAAGGTCC TCGCAGTTCCGC CTTTGACGAC GGGATGGAAA ACCCCTTCCT CCCCCACAAC  1140
TACCCCTCCCC ACGGCCCGGGT GGTCGGTCTAC ACCGGCACCC ACGACAACGA CACCACCCCTG  1200
GGCTGGTACC GCACGGCCAC CCCCCCACGAG AAGGCCTTCA TGCCGCCGGTA CCTGCCCGGAC  1260
TCCGCCGATCA CCTTCCGGGA AGAGGAGGAG GTGCCCCTGGG CCCTGATGCA CCTGGGGATG  1320
AAGTCCGTCGG CCCCGCTCGC CGTCTACCCCG GTGCAGGACG TCCTGGCCCT GGGCAGCGAG  1380
GCCCCGGATGA ACTACCCCGG AAGGCCCTCG GGGAACTGGG CCTGGCCGCT CCTCCCCCGGG  1440
GAGCTTTCCC CGGAGCACGG GGCGAGGCTT AGCGCCCATGG CCGAGGCCAC GCAACGGCTC  1500
TAG                                                               1503
```